(19) Europäisches Patentamt
European Patent Office
Office européen des brevets

(11) EP 2 666 460 A1

(12) **EUROPEAN PATENT APPLICATION**
published in accordance with Art. 153(4) EPC

(43) Date of publication:
**27.11.2013 Bulletin 2013/48**

(21) Application number: **11856236.2**

(22) Date of filing: **20.01.2011**

(51) Int Cl.:
***A61K 8/49*** *(2006.01)* ***A61Q 19/02*** *(2006.01)*

(86) International application number:
**PCT/JP2011/050986**

(87) International publication number:
**WO 2012/098664 (26.07.2012 Gazette 2012/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Nichirei Biosciences Inc.**
**Tokyo 104-8402 (JP)**

(72) Inventors:
- **NAGAMINE Kenichi**
**Tokyo 104-8402 (JP)**
- **KAWAGUCHI Masakazu**
**Higashimurayama-shi**
**Tokyo 189-0003 (JP)**
- **HIMONO Ayaka**
**Higashimurayama-shi**
**Tokyo 189-0003 (JP)**

(74) Representative: **Cabinet Plasseraud**
**52, rue de la Victoire**
**75440 Paris Cedex 09 (FR)**

# (54) SKIN-WHITENING AGENT CONTAINING 3-HYDROXY-2-PYRONE

(57) This invention provides a skin-whitening agent, a tyrosinase activity inhibitor, and melanin production inhibitor comprising, as an active ingredient, a compound exhibiting excellent solubility in various solvents. Such skin-whitening agent, tyrosinase activity inhibitor, and melanin production inhibitor comprise 3-hydroxy-2-pyrone.

Fig. 2

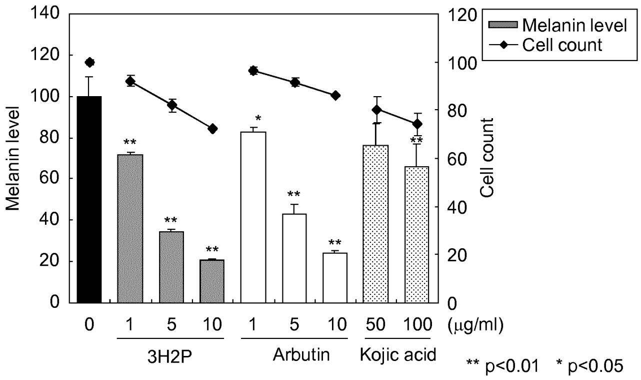


EP 2 666 460 A1

## Description

### Technical Field

The present invention relates to the use of 3-hydroxy-2-pyrone for a skin-whitening agent, a tyrosinase activity inhibitor, and a melanin production inhibitor.

### Background Art

In general, pigmentation on the skin, such as blemishes, is considered to occur upon activation of melanocytes caused by ultraviolet stimulation and enhanced biosynthesis of melanin pigments. Accordingly, many substances that suppress the biosynthesis of melanin pigments have been developed as skin-whitening agents.

It is well known that ascorbic acid has skin-whitening effects for inhibiting melanin synthesis in cells due to the inhibition of tyrosinase activity. However, ascorbic acid disadvantageously is degraded in an aqueous solution and causes coloration, such as browning. In order to overcome such disadvantages, many ascorbic acid derivatives that are more stable in aqueous solutions have been developed. Such derivatives are compounds resulting from esterification of ascorbic acid via introduction of a phosphate group, fatty acid such as stearic acid, or sugar such as glucose into a hydroxyl group at position 2, 3, 5, or 6 of the ascorbic acid. Such derivatives are expected to function in such a manner that they would be absorbed by an organism, hydrolyzed with enzymes to generate ascorbic acid, and thereby exert skin-whitening effects in an organism. These derivatives, however, are problematic in terms of solubility in various solvents, and, disadvantageously, they do not function without being hydrolyzed.

As other substances exerting skin-whitening effects, for example, hydroquinone derivatives, kojic acid and derivatives thereof, and pyrone compounds and derivatives thereof are known (Patent Documents 1 and 2). However, these substances were problematic in terms of solubility in various solvents, skin-whitening effects, and other properties. Therefore, the development of skin-whitening agents that can exert excellent solubility in various solvents and skin-whitening effects has been desired.

3-Hydroxy-2-pyrone (hereafter it may occasionally be referred to as "3H2P") is an oxidative degradation product of ascorbic acid, which is reported in Non-Patent Document 1. While it is well known that ascorbic acid is converted into dehydroascorbic acid and diketoglucuronic acid during the process of oxidation, further-oxidized substances are not very well known.

Patent Document 3 and Non-Patent Document 2 describe methods for obtaining 3H2P from ascorbic acid in the form of an oxidative degradation product.

The functions of 3H2P are not very well known, and only a small number of reports have been made concerning 3H2P. Patent Document 3 describes that 3H2P is an amphipathic antioxidant. It reports that 3H2P is excellent in terms of platelet aggregation inhibition and curing of hepatic disorders, and thus that it is usable for a pharmaceutical composition or functional food or beverage product.

### Prior Art Documents

#### Patent Documents

Patent Document 1: JP S53-3538 A (1978)
Patent Document 2: JP H04-2562 A (1992)
Patent Document 3: JP 2007-246475 A

#### Non-Patent Documents

Non-Patent Document 1: Bioscience, Biotechnology, and Biochemistry, 69 (11), pp. 2129-2137, 2005
Non-Patent Document 2: N. Morita et al., Bull. Univ. Osaka Pref., Ser. B, Vol. 41, pp. 61-69, 1989

Summary of the Invention

Problem to be Solved by the Invention

The present invention provides a skin-whitening agent, a tyrosinase activity inhibitor, and a melanin production inhibitor comprising, as an active ingredient, a compound exhibiting excellent solubility in various solvents.

Means for Solving the Problem

3-Hydroxy-2-pyrone (3H2P) is a compound exhibiting excellent solubility in various solvents. 3H2P has a chemical structure represented by the formula:

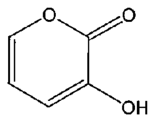

3H2P is represented by a molecular formula $C_5H_4O_3$. The molecular weight thereof is 112.084, its CAS Registry Number is 496-64-0, its systematic name is 3-hydroxy-2-pyrone or 3-hydroxy-2H-pyran-2-one, and its common name is isopyromucic acid. However, no skin-whitening effects of 3H2P were known in the past.

The present inventors discovered that 3H2P has remarkably strong inhibitory effects on tyrosinase activity and inhibitory effects on melanin production and thus is a useful skin-whitening substance. This has led to the completion of the present invention. The present invention includes the following inventions.

(1) A skin-whitening agent comprising 3-hydroxy-2-pyrone.
(2) A tyrosinase activity inhibitor comprising 3-hydroxy-2-pyrone.
(3) A melanin production inhibitor comprising 3-hydroxy-2-pyrone.
(4) A composition for an external skin preparation comprising 3-hydroxy-2-pyrone and an ingredient that is approved for application to the skin.

The present invention further includes the following inventions.

(5) A skin-whitening method comprising a step of administration of 3-hydroxy-2-pyrone to a subject, such as a human, who desires skin whitening. Such method may be implemented for a medical or cosmetic purpose.
(6) 3-Hydroxy-2-pyrone for skin-whitening.
(7) Use of 3-hydroxy-2-pyrone for the production of an external preparation for skin-whitening.
(8) Use of 3-hydroxy-2-pyrone for the production of a medicament for skin-whitening.
(9) A method for inhibition of tyrosinase activity *in vivo* or *in vitro* comprising a step of addition of 3-hydroxy-2-pyrone to tyrosinase *in vivo* or *in vitro.* The method for inhibition of tyrosinase activity *in vivo* may be implemented for a medical or cosmetic purpose.
(10) A method for prevention or treatment of a disease or symptom that is relieved by the inhibition of tyrosinase activity, comprising a step of administration of 3-hydroxy-2-pyrone to a subject, such as a human, who is in need of prevention or treatment of such disease or symptom.
(11) 3-Hydroxy-2-pyrone used for the inhibition of tyrosinase activity *in vivo* or *in vitro.*
(12) Use of 3-hydroxy-2-pyrone for the production of an external skin preparation for the inhibition of tyrosinase activity.
(13) Use of 3-hydroxy-2-pyrone for the production of a medicament for prevention or treatment of a disease or symptom that is relieved by the inhibition of tyrosinase activity.
(14) A method for inhibition of melanin pigment production *in vivo* or *in vitro* comprising a step of addition of 3-hydroxy-2-pyrone to a cell of an animal, such as a human, *in vivo* or *in vitro.* The method for inhibition of melanin pigment production *in vivo* may be implemented for a medical or cosmetic purpose.
(15) A method for prevention or treatment of a disease or symptom that is relieved by the inhibition of melanin pigment production, comprising a step of administration of 3-hydroxy-2-pyrone to a subject, such as a human, who is in need of prevention or treatment of such disease or symptom.
(16) 3-Hydroxy-2-pyrone used for the inhibition of melanin pigment production in a cell *in vivo* or *in vitro.*
(17) Use of 3-hydroxy-2-pyrone for the production of an external skin preparation for the inhibition of melanin production.
(18) Use of 3-hydroxy-2-pyrone for the production of a medicament for prevention or treatment of a disease or

symptom that is relieved by the inhibition of melanin pigment production in a cell.

Effects of the Invention

The present invention provides a skin-whitening agent, a tyrosinase activity inhibitor, and a melanin production inhibitor comprising, as an active ingredient, 3-hydroxy-2-pyrone, which is a compound exhibiting excellent solubility in various solvents.

Brief Description of the Drawings

Fig. 1 shows inhibitory effects of kojic acid, ascorbic acid, and 3-hydroxy-2-pyrone (3H2P) on tyrosinase activity measured in Experiment 2.
Fig. 2 shows inhibitory effects of 3-hydroxy-2-pyrone (3H2P), arbutin, and kojic acid on melanin production measured in Experiment 3.
Fig. 3 shows inhibitory effects of kojic acid and $\alpha$-pyrones on tyrosinase activity measured in Experiment 4.
Fig. 4 shows inhibitory effects of coumalin ($\alpha$-pyrone), coumalic acid, and 3-hydroxy-2-pyrone (3H2P) on tyrosinase activity measured in Experiment 5.
Fig. 5 shows inhibitory effects of kojic acid, 3-hydroxy-2-pyrone (3H2P), and 3-hydroxy-4-pyrone (3H4P) on tyrosinase activity measured in Experiment 6.
Fig. 6 shows inhibitory effects of kojic acid, 3-hydroxy-2-pyrone (3H2P), and 3-hydroxy-4-pyrone (3H4P) on melanin production measured in Experiment 7.

Embodiments for Carrying out the Invention

3-Hydroxy-2-pyrone (3H2P) used in the present invention can be synthesized from dehydroascorbic acid in accordance with the procedure described in, for example, Patent Document 3.

It is not necessary that 3H2P be used in an isolated and purified state. 3H2P is contained in natural products containing ascorbic acid (e.g., acerola) as an oxidative degradation product of ascorbic acid. A natural composition containing 3H2P (e.g., acerola) or a non-natural composition resulting from treatment of such natural composition, so as to adjust the 3H2P concentration to be higher than that of the general natural composition (with 3H2P concentration preferably being at least 0.001%, and more preferably 0.01% to 1.7% by weight, relative to the total amount of the composition) can be used for the present invention.

3H2P may be used in the form of a solvate, such as a hydrate. When 3H2P is in the form of a solvate, it is preferably a physiologically acceptable solvate (i.e., a solvate that is approved for use in a pharmaceutical, cosmetic, food or beverage, or other form of product).

3H2P has inhibitory effects on tyrosinase activity and inhibitory effects on melanin production in a cell. 3H2P exerts skin whitening effects due to such effects.

Through administration of 3H2P to a subject, such as a human, who desires skin-whitening, skin-whitening can be realized (i.e., skin damage caused by pigmentation, such as blemishes or freckles, can be inhibited or relieved). According to such technique, 3H2P is administered to a subject before pigmentation on the skin occurs due to sunburn, so as to prevent the pigmentation or suppress the progression thereof. Alternatively, 3H2P may be administered to a subject who has already experienced pigmentation, so as to relieve the skin damage caused by pigmentation. Such administration may be implemented for a cosmetic purpose, or it may be implemented by a physician for a medical purpose.

3H2P can be used for a method for the inhibition of tyrosinase activity *in vivo* or *in vitro.* The term "inhibition of tyrosinase activity *in vivo"* refers to the inhibition of tyrosinase activity in an organism, such as a human (and inhibition of tyrosinase activity in skin cells, in particular). The method for the inhibition of tyrosinase activity *in vivo* may be implemented for a cosmetic purpose, or it may be implemented by a physician for a medical purpose.

3H2P can be used for the prevention or treatment of a disease or symptom that is relieved by the inhibition of tyrosinase activity. Examples of such disease or symptom include blemishes and freckles.

3H2P can be used for the method for the inhibition of melanin production *in vivo* or *in vitro.* The term "inhibition of melanin production *in vivo"* refers to the inhibition of melanin pigment production in an organism, such as a human (and inhibition of melanin production in skin chromocytes, in particular). The method for the inhibition of melanin production *in vivo* may be implemented for a cosmetic purpose, or it may be implemented by a physician for a medical purpose.

3H2P can be used for the prevention or treatment of a disease or symptom that is relieved by the inhibition of melanin production. Examples of such disease or symptom include blemishes and freckles.

The forms of 3H2P administration to a subject, such as a human, for the purpose of skin-whitening or for the purpose of inhibition of tyrosinase activity or melanin production *in vivo* are not particularly limited. For example, 3H2P may be administered to the subject via percutaneous administration involving direct application of 3H2P to the target skin (for example, in the form of the external skin preparations described below), oral administration (for example, in the form of powder, tablets, granules, fine grains, liquid, capsules, pills, troches, peroral liquid preparations, suspensions, emulsions, syrups, or elixirs), or parenteral administration other than percutaneous administration (for example, in the form of injection preparations, instillation, suppositories, inhalants, or transmucosal absorbents).

3H2P can be incorporated into an external skin preparation, such as a cosmetic product, quasi-drug, or pharmaceutical product, together with an ingredient that is approved for application to the skin (e.g., other ingredients exhibiting skin-whitening effects, other active ingredients, or ingredients used for the production of preparations), as an active ingredient that exhibits skin-whitening effects, inhibitory effects on tyrosinase activity, or inhibitory effects on melanin production. 3H2P content in the external skin preparation is not particularly limited, provided that 3H2P is able to effectively exhibit skin-whitening effects, inhibitory effects on tyrosinase activity, and/or inhibitory effects on melanin production. Based on the results attained in Experiment 3 and the amounts of active ingredients that can be incorporated into a general external skin preparation, it is preferable that 3H2P content be between 0.0001% and 5% by weight, based on the total amount of the composition for the external skin preparation.

Examples of other ingredients exhibiting skin-whitening effects that can be incorporated into an external skin preparation include ascorbic acid, an ascorbic acid derivative (e.g., a salt of ascorbic acid phosphate ester, a salt of ascorbic acid sulfate ester, ascorbic acid glucoside, or ethyl ascorbic acid), a γ-pyrone glycoside, γ-pyrones such as kojic acid and a kojic acid derivative, and a hydroquinone derivative such as arbutin.

An external skin preparation can be prepared with the use of ingredients that are generally used for the production of cosmetic products, quasi-drugs, or pharmaceutical products. Examples of such ingredients include purified water, alcohols, oil substances, moisturizers, thickeners, preservatives, emulsifiers, crude drug components, ultraviolet absorbers, pigments, aromas, emulsion stabilizers, and pH regulators.

The form of an external skin preparation is not limited, and it may be, for example, a cream, ointment, emulsion, lotion, solution, gel, facial mask, or stick.

## Examples

### Experiment 1: Preparation of 3H2P

Purified water (50 ml) was added to 5.0268 g of dehydroascorbic acid (an oxidation product of ascorbic acid, Wako Pure Chemical Industries, Ltd.) and the mixture was heated at 100°C for 3 hours to obtain a heat-treated solution.

The resulting heat-treated solution was subjected to extraction with ethyl acetate. The ethyl acetate layer was collected, anhydrous sodium sulfate was added thereto, and the resultant was refrigerated overnight for dehydration. The ethyl acetate layer was concentrated using a rotary evaporator to obtain a fluid concentrate. The fluid concentrate was introduced into a watch glass, a beaker was placed upside-down over the watch glass, the watch glass was heated at 80°C for 8 hours, and the sublimed components were allowed to adhere to the inside of the beaker. The resultant was dried to collect 293.7 mg of the crystalline sublimate. As a result of analysis of the sublimate by high-performance liquid chromatography (HPLC), the presence of 3-hydroxy-2-pyrone (3H2P) together with 2-furancarboxylic acid (2FA) was observed.

In order to eliminate 2FA, the resulting sublimate (293.7 mg) was dissolved in 8 ml of 0.05% HCl-containing purified water, and the resultant was applied to Sep-Pak C18 (Waters) and eluted with 0.05% HCl-containing purified water and a 0.05% HCl-containing 10% methanol solution, in order to collect a 3H2P-containing fraction. The fraction was concentrated using a rotary evaporator and subjected to extraction with ethyl acetate to collect the ethyl acetate layer. Anhydrous sodium sulfate was added thereto, the mixture was refrigerated for dehydration, the fluid concentrate obtained by evaporation distillation was introduced into a watch glass, a beaker was placed upside-down over the watch glass, the watch glass was heated at 80°C for 3 hours, and the sublimed 3H2P was allowed to adhere to the inside of the beaker. The resultant was dried to collect 139.9 mg of the crystalline 3H2P. The resulting sublimate was analyzed by HPLC and confirmed to be high-purity 3H2P.

### Experiment 2: Measurement of inhibitory effects on tyrosinase activity

Tyrosinase is known to be an important enzyme that acts when a melanin pigment is produced from tyrosine as an amino acid. Many skin-whitening substances have the inhibitory effects on tyrosinase activity. Thus, the inhibitory effects of kojic acid, ascorbic acid, and 3H2P prepared in Experiment 1 on tyrosinase activity were measured.

In the experiment, 0.2 ml of an enzyme solution containing 72 units/ml tyrosinase (Sigma) was mixed with 1.0 ml of a substrate mixture (i.e., a 2:2:1 mixture of a 6 mM L-DOPA solution, the test composition with the adjusted

concentration (hereafter referred to as the "sample"), and phosphate buffer), the absorbance was measured at 475 nm at 37°C at 0 min and 3 min, and the inhibition rate of tyrosinase activity was determined using the equation shown below.

Inhibition rate of tyrosinase activity (%) = (1 - (the difference in the absorbance of the sample between 0 min and 3 min) / (the difference in the absorbance of the blank between 0 min and 3 min) x 100

The results are shown in Fig. 1. The measurement demonstrates that the inhibitory effects of 3H2P on tyrosinase activity are equivalent to those of ascorbic acid, although such effects are somewhat weaker than those of kojic acid.

Experiment 3: Inhibition of melanin production

Since the inhibitory effects of 3H2P on tyrosinase activity were confirmed in Experiment 2, inhibitory effects on melanin production in animal cells were further examined. 3H2P prepared in Experiment 1, arbutin, and kojic acid were employed as samples.

B16 mouse melanoma 4A5 cells (RIKEN) were seeded at $5.0 \times 10^4$ cells/well on a 6-well plate and cultured in Dulbecco's modified Eagle's medium (DMEM) supplemented with 10% FBS at 37°C in the presence of 5% $CO_2$. The medium was exchanged with fresh medium supplemented with the sample on the following day, and culture was conducted in the medium supplemented with the sample for an additional 5 days. The medium was removed 6 days after the initiation of culture, and the cells were collected by using trypsin. The collected cells were suspended in 1N sodium hydroxide, and melanin was extracted via heating. The absorbance of the melanin extract was measured at 405 nm, and the melanin level was quantified with the calibration curve of the melanin reagent (Sigma). In addition, the amount of proteins in the collected cells was determined using the DC Protein Assay KIT (BIO-RAD) and used as the indicator for the viable cell count. Through such measurement, the melanin level and the cell count were determined relative to levels observed in the control not supplemented with the sample, designated as 100. The results are shown in Table 1 and Fig. 2. The results demonstrate that the inhibitory effects of 3H2P on melanin production were stronger than those of kojic acid and equivalent to those of arbutin. Since 3H2P exerts inhibitory effects on melanin production at concentrations as low as 1 ppm (1 μg/ml, 0.0001%), 3H2P is considered to be a strong skin-whitening agent.

Table 1

| | (μg/ml) | Melanin level | Cell count |
|---|---|---|---|
| Blank | 0 | 100 | 100 |
| 3H2P | 1 | 71.7 | 92.2 |
| | 5 | 34.3 | 81.9 |
| | 10 | | 72.4 |
| Arbutin | 1 | 82.4 | 96.2 |
| | 5 | 43.0 | 91.7 |
| | 10 | 23.9 | 86.0 |
| Kojic acid | 50 | 76.5 | 80.2 |
| | 100 | 65.7 | 74.1 |

Experiment 4: Comparison of inhibitory effects of α-pyrones on tyrosinase activity (1)

The inhibitory effects of 3-hydroxy-2-pyrone on tyrosinase activity were compared with those of kojic acid as a γ-pyrone compound, or of coumalin and coumalic acid as α-pyrone compounds. The inhibitory effects of kojic acid, coumalin, and coumalic acid on melanin production are reported in Patent Document 1.

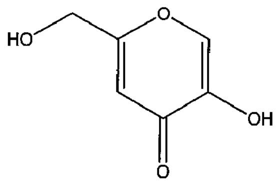 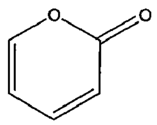 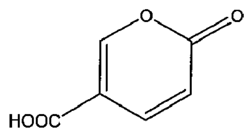

Kojic acid Coumalin (α-pyrone) Coumalic acid

The experiment was carried out in the same manner as that described in Patent Document 1.

An aqueous solution of 1% 3H2P, an aqueous solution of 1% coumalin (Tokyo Chemical Industry Co., Ltd.), an aqueous solution of 1% coumalic acid (Tokyo Chemical Industry Co., Ltd.), and an aqueous solution of 0.01% kojic acid were prepared. Subsequently, 1.45 ml of a substrate-sample mixture comprising an aqueous solution of L-tyrosine (0.3 mg/ml), a sample solution of 3H2P, etc., and McIlvaine buffer (pH 6.8) was introduced into a test tube, 0.05 ml of a tyrosinase solution (1 mg/ml) was added thereto, the resultant was mounted on a spectrophotometer capable of maintaining a constant temperature of 37°C, and the absorbance was measured at 475 nm with the elapse of time (once every minute between 0 min and 15 min). The results are shown in Fig. 3.

A blank solution to which purified water ($H_2O$) was added did not contain a substance that would inhibit tyrosinase activity. Thus, the amount of dopachrome rapidly increased during the period up to 2 minutes after the initiation of the reaction. In contrast, insignificant levels of inhibitory effects were observed with the aqueous solution of coumalin, and strong inhibitory effects were observed with the aqueous solution of coumalic acid. Under the present conditions, the inhibitory effects of the aqueous solution of 3H2P on tyrosinase activity were significantly stronger than those of coumalin and coumalic acid.

Experiment 5: Comparison of inhibitory effects of α-pyrones on tyrosinase activity (2)

The inhibitory effects of coumalin (α-pyrone, Tokyo Chemical Industry Co., Ltd.), coumalic acid (Tokyo Chemical Industry Co., Ltd.), and 3H2P on tyrosinase activity were examined.

In the experiment, 0.2 ml of an enzyme solution containing 72 units/ml tyrosinase (Sigma) was mixed with 1.0 ml of a substrate mixture (i.e., a 2:2:1 mixture of a 6 mM L-DOPA solution, the sample with the adjusted concentration, and phosphate buffer (pH 6.8)), the absorbance was measured at 475 nm at 37°C at 0 min and 3 min, and the inhibition rate of tyrosinase activity was determined using the equation shown below.

Inhibition rate of tyrosinase activity (%) = (1 - (the difference in the absorbance of the sample between 0 min and 3 min) / (the difference in the absorbance of the blank between 0 min and 3 min) x 100

The samples were subjected to the experiment at the final concentrations of 0.3%, 0.1%, and 0.033%, and the inhibitory effects thereof on tyrosinase activity were compared. As a result, the inhibitory effects of 3H2P were found to be apparently stronger than those of coumalin and coumalic acid. The results are shown in Fig. 4.

Experiment 6: Comparison of inhibitory effects of 3H2P on tyrosinase activity with those of 3-hydroxy-4-pyrone

3-Hydroxy-4-pyrone, which has already been reported to have the skin-whitening effects (hereafter referred to as "3H4P," it is also referred to as, for example, pyromeconic acid or pyrocomenic acid), is represented by the same molecular formula as 3H2P, although it is a compound classified as a γ-pyrone. A 3H4P reagent manufactured by Molekula was used.

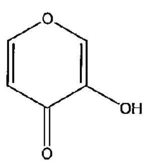

3H4P

In the experiment, 0.2 ml of an enzyme solution containing 72 units/ml tyrosinase (Sigma) was mixed with 1.0 ml of a substrate mixture (i.e., a 2:2:1 mixture of a 6 mM L-DOPA solution, the sample with the adjusted concentration, and phosphate buffer), the absorbance was measured at 475 nm at 37°C at 0 min and 3 min, and the inhibition rate of tyrosinase activity was determined using the equation shown below.

Inhibition rate of tyrosinase activity (%) = (1 - (the difference in the absorbance of the sample between 0 min and 3 min) / (the difference in the absorbance of the blank between 0 min and 3 min) x 100

As a result of the measurement, kojic acid (i.e., a γ-pyrone) was found to exhibit the strongest inhibitory effects on tyrosinase activity, and the inhibitory effects of 3H2P on tyrosinase activity were found to be equivalent to those of 3H4P (Fig. 5).

Experiment 7: Comparison of inhibitory effects of 3H2P on melanin production with those of 3H4P

Inhibitory effects of 3H4P, 3H2P, and kojic acid on melanin production were compared. 3H4P manufactured by Molekula was used.

The experiment was carried out in the following manner.

B16 mouse melanoma 4A5 cells (RIKEN) were seeded at $5.0 \times 10^4$ cells/well on a 6-well plate and cultured in Dulbecco's modified Eagle's medium (DMEM) supplemented with 10% FBS at 37°C in the presence of 5% $CO_2$. The medium was exchanged with fresh medium supplemented with the sample on the following day, and culture was conducted in the medium supplemented with the sample for an additional 5 days. The medium was removed 6 days after the initiation of culture, and the cells were collected by using trypsin. The collected cells were suspended in 1N sodium hydroxide, and melanin was extracted via heating. The absorbance of the melanin extract was measured at 405 nm, and the melanin level was quantified with the calibration curve of the melanin reagent (Sigma). In addition, the amount of proteins in the collected cells was determined using the DC Protein Assay KIT (BIO-RAD) and used as the indicator for the viable cell count. Through such measurement, the melanin level and the cell count were determined relative to levels observed in the control not supplemented with the sample, designated as 100. The results are shown in Table 2 and Fig. 6.

Table 2

| | (μg/ml) | Melanin level | Cell count |
|---|---|---|---|
| Blank | 0 | 100 | 100 |
| 3H2P | 1 | 77.2 | 102.0 |
| | 5 | 42.7 | 98.7 |
| | 10 | 29.6 | 93.8 |
| 3H4P | 50 | 88.8 | 89.7 |
| | 100 | 50.9 | 84.4 |
| Kojic acid | 50 | 85.6 | 80.2 |
| | 100 | 65.3 | 74.1 |

As a result, the inhibitory effects of 3H4P on melanin production were found to be equivalent to those of kojic acid, and 3H2P was found to have stronger inhibitory effects on melanin production than such γ-pyrone compounds.

## Claims

1. A skin-whitening agent comprising 3-hydroxy-2-pyrone.

2. A tyrosinase activity inhibitor comprising 3-hydroxy-2-pyrone.

3. A melanin production inhibitor comprising 3-hydroxy-2-pyrone.

4. A composition for an external skin preparation comprising 3-hydroxy-2-pyrone and an ingredient, which is approved for application to the skin.

Fig. 1

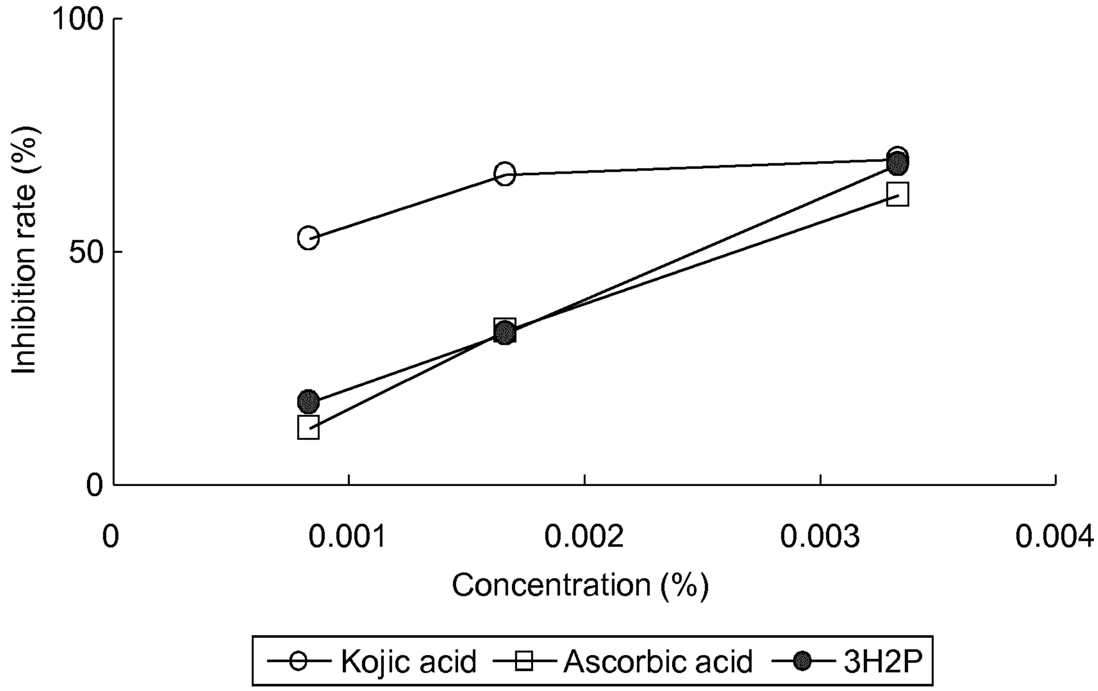
100
50
0
Inhibition rate (%)
0
0.001
0.002
0.003
0.004
Concentration (%)
Kojic acid
Ascorbic acid
3H2P

Fig. 2

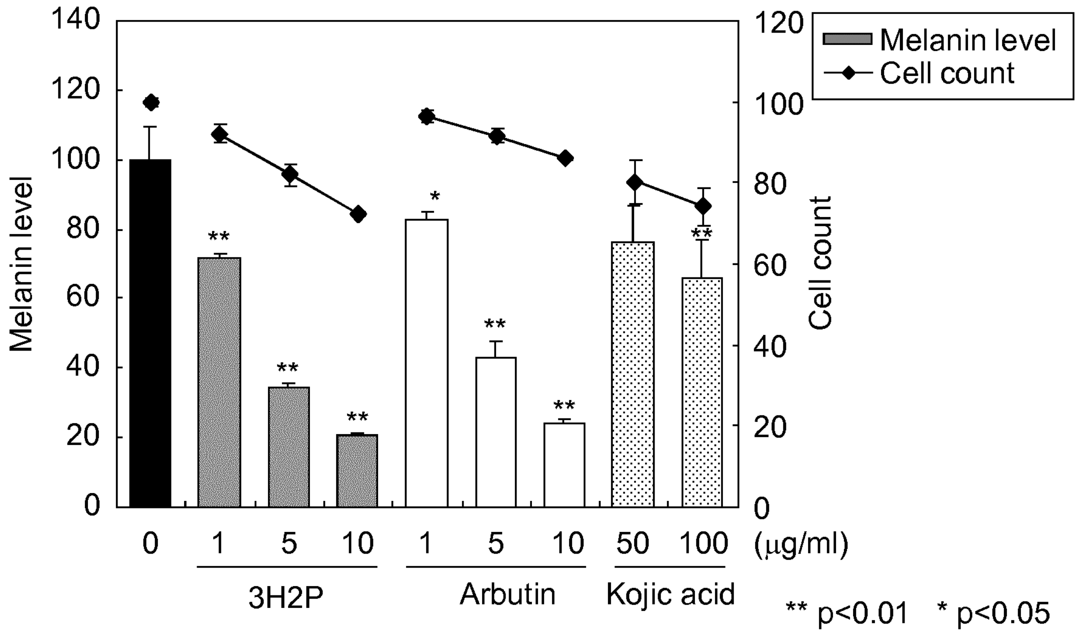
Melanin level
Cell count
140
120
100
80
60
40
20
0
120
100
80
60
40
20
0
Melanin level
Cell count
**
**
**
*
**
**
**
0
1
5
10
1
5
10
50
100
(μg/ml)
3H2P
Arbutin
Kojic acid
** p<0.01 * p<0.05

Fig. 3

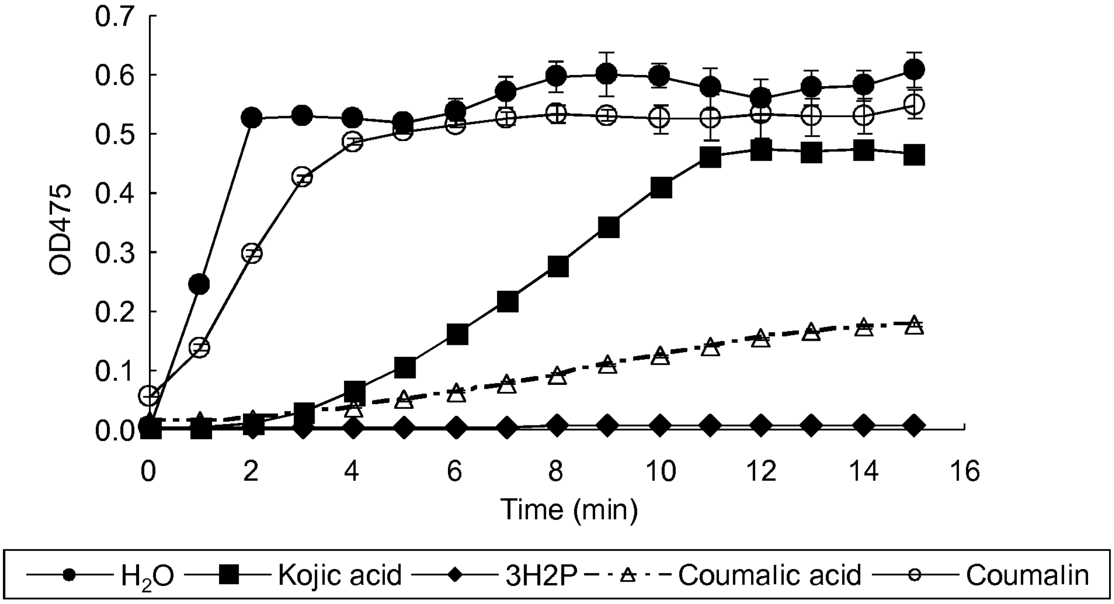
0.7
0.6
0.5
0.4
0.3
0.2
0.1
0.0
OD475
0
2
4
6
8
10
12
14
16
Time (min)
H2O
Kojic acid
3H2P
Coumalic acid
Coumalin

Fig. 4

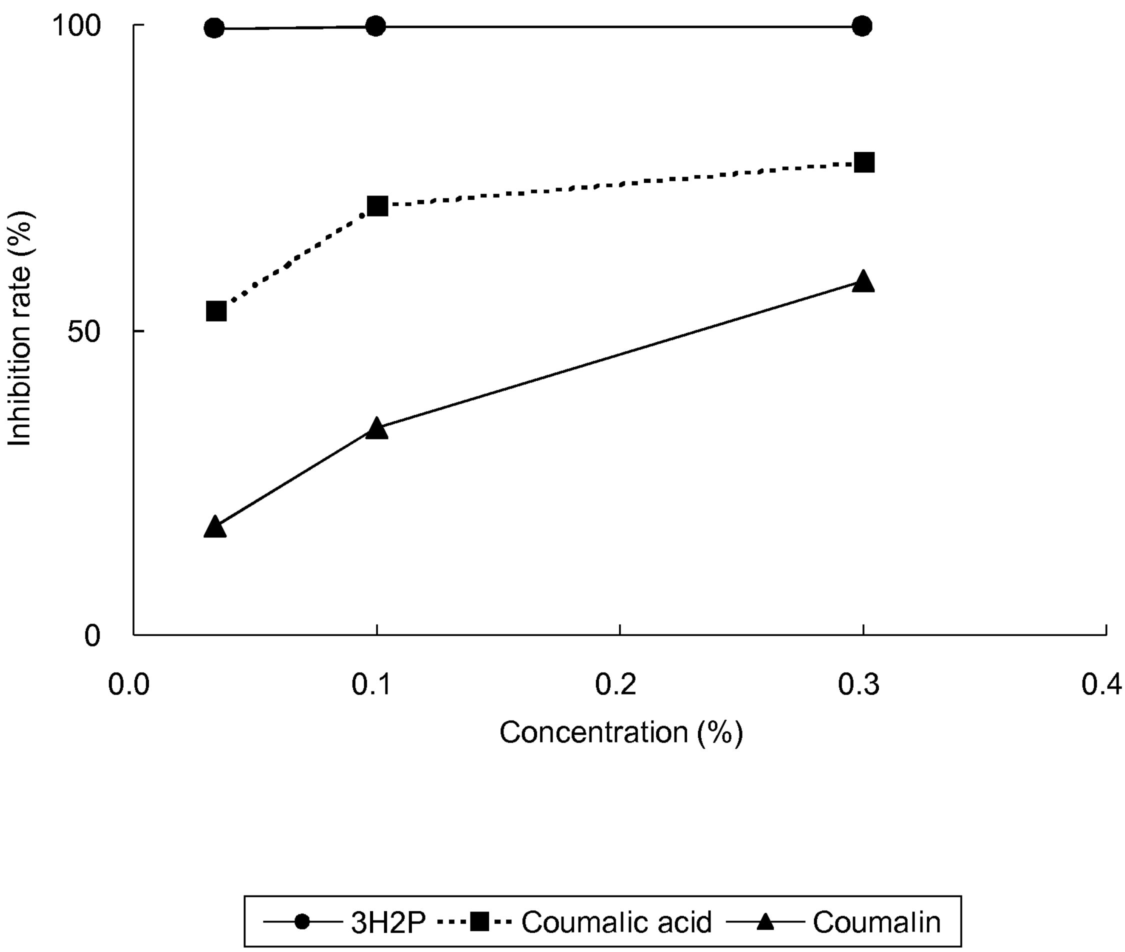
100
50
0
Inhibition rate (%)
0.0
0.1
0.2
0.3
0.4
Concentration (%)
3H2P
Coumalic acid
Coumalin

Fig. 5

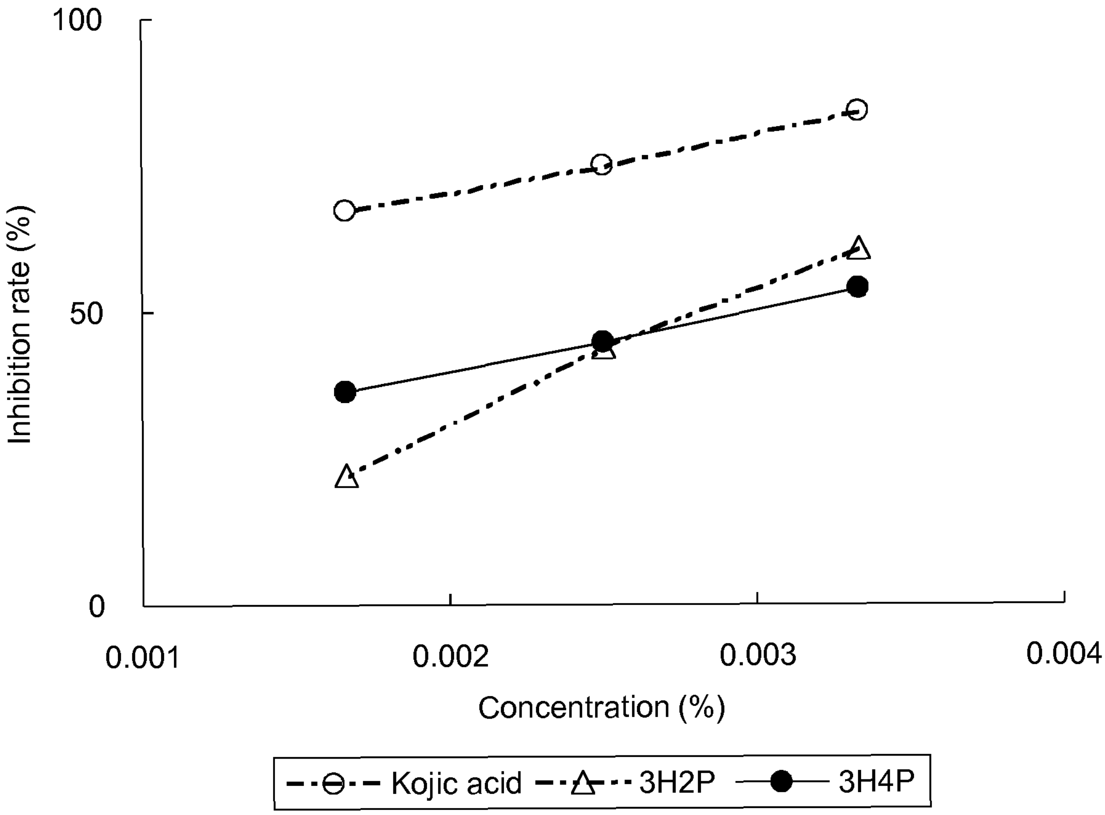
100
50
0
Inhibition rate (%)
0.001
0.002
0.003
0.004
Concentration (%)
Kojic acid
3H2P
3H4P

Fig. 6

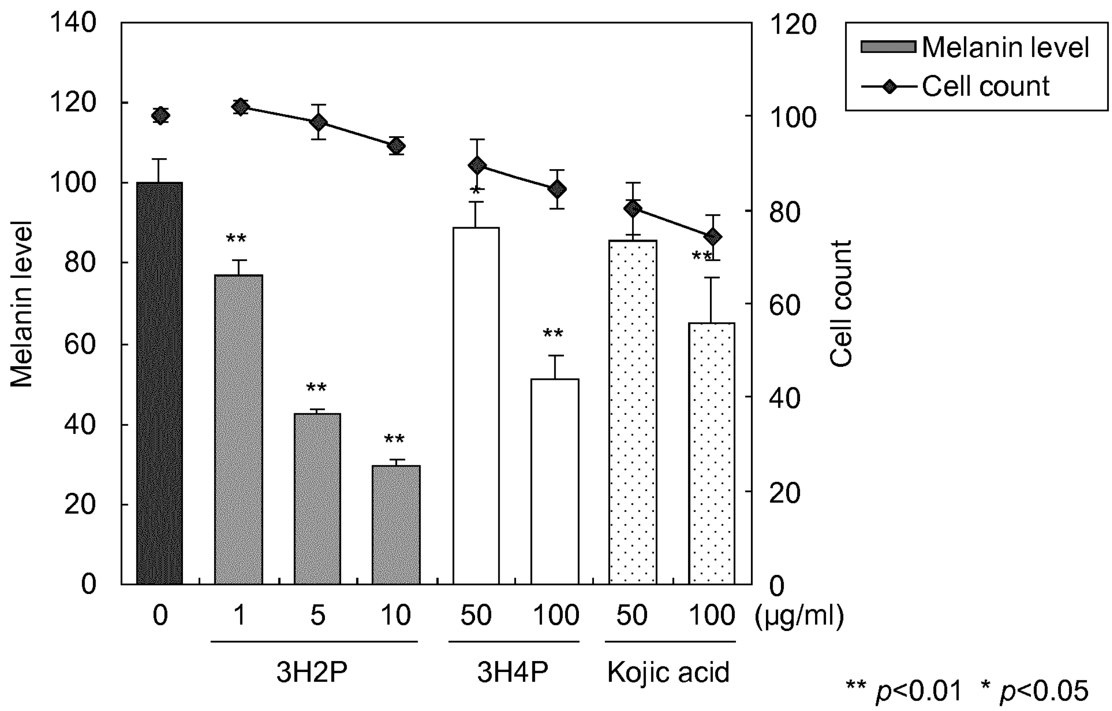
140
120
100
80
60
40
20
0
Melanin level
120
100
80
60
40
20
0
Cell count
Melanin level
Cell count
**
**
**
*
**
**
0
1
5
10
50
100
50
100
(μg/ml)
3H2P
3H4P
Kojic acid
** p<0.01 * p<0.05

**INTERNATIONAL SEARCH REPORT**

International application No.
PCT/JP2011/050986

A. CLASSIFICATION OF SUBJECT MATTER
*A61K8/49*(2006.01)i, *A61Q19/02*(2006.01)i

According to International Patent Classification (IPC) or to both national classification and IPC

B. FIELDS SEARCHED

Minimum documentation searched (classification system followed by classification symbols)
A61K8/49, A61Q19/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 3-47899 A (Ogawa & Co., Ltd.),<br>28 February 1991 (28.02.1991),<br>claims; page 1, left column<br>(Family: none) | 4<br>1-3 |
| A | WO 2008/153784 A2 (EASTMAN CHEMICAL CO.),<br>18 December 2008 (18.12.2008),<br>& JP 2010-528654 A & US 2008/0306144 A1<br>& EP 2155886 A1 & CN 101680008 A | 1-4 |
| A | JP 59-33207 A (Sansho Seiyaku Co., Ltd.),<br>23 February 1984 (23.02.1984),<br>(Family: none) | 1-4 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 February, 2011 (02.02.11) | 15 February, 2011 (15.02.11) |
| Name and mailing address of the ISA/<br>Japanese Patent Office<br>Facsimile No. | Authorized officer<br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S533538 A
- JP H042562 A
- JP 2007246475 A


### Non-patent literature cited in the description


- *Bioscience, Biotechnology, and Biochemistry,* 2005, vol. 69 (11), 2129-2137
- **N. MORITA et al.** *Bull. Univ. Osaka Pref., Ser. B,* 1989, vol. 41, 61-69